**Europäisches Patentamt**

**European Patent Office**

**Office européen des brevets**

(11) Veröffentlichungsnummer: **0 515 994 A2**

## EUROPÄISCHE PATENTANMELDUNG

(21) Anmeldenummer: **92108654.2**

(22) Anmeldetag: **22.05.92**

(51) Int. Cl.5: **B01J 2/18**, C12M 1/40

(30) Priorität: **25.05.91 DE 4117079**

(43) Veröffentlichungstag der Anmeldung:
**02.12.92 Patentblatt 92/49**

(84) Benannte Vertragsstaaten:
**CH DE ES FR GB IT LI NL**

(71) Anmelder: **B. BRAUN BIOTECH INTERNATIONAL GmbH**
**Schwarzenberger Weg 73-79**
**W-3508 Melsungen(DE)**

(72) Erfinder: **Kahlert, Wolfgang, Dipl.-Ing.**
**Akazienweg 1**
**W-3501 Körle(DE)**

(72) Erfinder: **Kuhlmann, Winfried, Dr.Dipl.-Chem.**
**Schulstrasse 12**
**W-3508 Melsungen(DE)**
Erfinder: **Rietschel, Wolfgang, Dipl.-Ing.**
**Obere Bergstrasse 2**
**W-3501 Söhrewald 1(DE)**
Erfinder: **Vorlop, Klaus D., Prof. Dr.Dipl-Chem.**
**Hochstrasse 7**
**W-3300 Braunschweig(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) **Vorrichtung zur Herstellung von Biokatalysatorperlen.**

(57) In einem Auffanggefäß (10), das eine Vernetzerlösung oder ein Umfällbad enthält, ist eine Tropfvorrichtung (16) angebracht, mit Auslaufrohren (46), aus denen ein Gemisch aus Trägerflüssigkeit und Biokatalysator austritt. In einer Druckkammer (44) befindet sich Druckgas, das durch die Auslaufrohre (46) umgebende Öffnungen austritt und die austretende Flüssigkeit zertropfen läßt. Die Tropfen fallen in die Vernetzerlösung und erstarren dort, wodurch Feststoff-Katalysatorperlen gebildet werden. Die Tropfvorrichtung (16) ist in einem Kopfstück (15) enthalten, das ein Rohr (14) aufweist, welches in eine relativ enge Öffnung der Oberwand (11) des Auffanggefäßes (10) eingesetzt werden kann. Die Behälteröffnung und das Rohr (14) können standardisiert werden, wobei unterschiedliche Kopfstücke (15) mit unterschiedlichen Zahlen von Auslaufrohren (46) bei demselben Auffanggefäß (10) benutzt werden können. Andererseits kann das gleiche Kopfstück (15) bei unterschiedlichen Auffanggefäßen benutzt werden. Die Vorrichtung kann in situ mit Dampf oder im Autoklaven sterilisiert werden. Eine Bypass-Leitung (40) ermöglicht auch das Sterilisieren der Zuführkammer (22).

FIG.1

Die Erfindung betrifft eine Vorrichtung der im Oberbegriff des Patentanspruchs 1 angegebenen und aus EP-0 140 336 B1 bekannten Art.

Immobilisierte Zellen sind wirksame Katalysatoren bei der Konversion organischer Bestandteile. Es ist bekannt, die Immobilisierung von Zellen durchzuführen, indem die Zellen zusammen mit einer Trägersubstanz in eine gelierfähige Polymerlösung eingegeben werden, die dann unter Tropfenbildung aus dem Immobilisierungsgefäß ausläuft und in ein Auffanggefäß fällt, das eine Vernetzerlösung enthält (J. Klein u. K.-D. Vorlop (1983) ACS Symposium Series 207, 377-392; American Chemical Society 1983, 17). Die Tropfen werden gebildet, indem koaxial zu den aus dem Immobilisierungsgefäß herausführenden Auslaufrohren sterile Druckluft zugeführt wird, die an den Auslaufrohren entlangstreicht und die Formung und Ablösung der Tropfen von dem Auslaufrohr erleichtert. Die Tropfen nehmen während des freien Falls und bei der Vernetzung Kugelform an, so daß in dem Auffanggefäß die Biokatalysatorperlen in Form von Kugeln vorhanden sind.

Von besonderer Bedeutung bei der Immobilisierung von Enzymen und Zellen ist die Sterilhaltung vor und während des Immobilisierungsprozesses. EP-0 140 336 B1 beschreibt eine Vorrichtung, die die Herstellung von Biokatalysatorperlen unter sterilen Bedingungen ermöglicht. Bei dieser Vorrichtung ist unmittelbar auf dem Auffanggefäß ein Immobilisierungsgefäß angebracht, das in einer Öffnung des Deckels des Auffanggefäßes befestigt ist und von dem sich die Auslaufrohre durch die Öffnung hindurch in das Auffanggefäß erstrecken. In das Immobilisierungsgefäß werden die Trägerflüssigkeit und der Biokatalysator getrennt voneinander eingegeben und im Immobilisierungsgefäß werden beide Komponenten miteinander vermischt. Das Mischen kann auch in einem externen Gefäß erfolgen, das mit dem Immobilisierungsgefäß über eine Schlauchleitung verbunden ist. Hierbei ist nur ein chargenweiser Betrieb möglich, wobei die Chargengröße von der Größe des Immobilisierungsgefäßes bzw. des externen Gefäßes abhängt. Ein wesentlicher Nachteil der bekannten Vorrichtung besteht darin, daß die Öffnung des Auffanggefäßes an die Größe des Fußbereichs des unmittelbar auf dem Auffanggefäß angebrachten Immobilisierungsgefäßes angepaßt sein muß. Das Immobilisierungsgefäß kann somit stets nur in Verbindung mit einem Auffanggefäß verwendet werden, dessen Deckelöffnung auf das Immobilisierungsgefäß abgestimmt ist. Ferner ist die die Auslaufrohre enthaltende Tropfvorrichtung fester Bestandteil des Immobilisierungsgefäßes. Es ist daher nicht möglich, bei einem Immobilisierungsgefäß die Anzahl der Auslaufrohre zu verändern. Die Anzahl der Auslaufrohre ist auch an die Größe der Deckelöffnung des Auffanggefäßes gebunden. Im allgemeinen wäre es günstig, die Deckelöffnung möglichst klein zu halten bzw. eine Deckelöffnung mit einer Normgröße von z.B. 19 mm Durchmesser zu verwenden. Eine derartig kleine Deckelöffnung reicht für den Durchtritt der Tropfvorrichtung normalerweise nicht aus.

Der Erfindung liegt das Problem zugrunde, eine Vorrichtung zur Herstellung von Biokatalysatorperlen zu schaffen, bei der die Größe der Tropfvorrichtung keine spezielle Anpassung an das Auffanggefäß bzw. dessen Öffnung erfordert.

Die Lösung dieses Problems erfolgt erfindungsgemäß mit den im Anspruch 1 angegebenen Merkmalen.

Bei der erfindungsgemäßen Vorrichtung ist die Tropfvorrichtung in einem Kopfstück enthalten, das ein Rohr aufweist, welches durch die Öffnung des Auffanggefäßes hindurchgesteckt wird. Damit ist es möglich, das Auffanggefäß mit einer relativ kleinen Öffnung zu versehen und in Verbindung hiermit eine Tropfvorrichtung zu schaffen, die eine größere Breite hat als die Öffnung. Das Kopfstück wird von innen her gegen die Oberwand bzw. den Deckel des Auffanggefäßes gesetzt, wobei das Rohr durch die Öffnung des Auffanggefäßes hindurchragt. Mit der Erfindung ist es möglich, eine Tropfvorrichtung mit einem Kopfstück, das eine große Breite bzw. einen großen Durchmesser hat, an einem Auffanggefäß anzubringen, welches eine kleine Öffnung aufweist. Die Öffnung kann beispielsweise eine Standardöffnung von 19 mm Durchmesser sein. Hinsichtlich der Größe des Kopfstücks besteht bei gleichbleibender Öffnung des Auffanggefäßes eine große Variationsbreite. So können Kopfstücke mit großer Breite und Kopfstücke mit kleinerer Breite wahlweise verwendet werden, wobei jedes dieser Kopfstücke den gleichen Durchmesser des zugehörigen Rohres aufweist.

Das von innen gegen den Deckel des Auffanggefäßes gesetzte Kopfstück wird zweckmäßigerweise mit einer Spannvorrichtung befestigt, die an dem Rohr angreift und das Kopfstück gegen den Deckel des Auffanggefäßes zieht. Über dem Kopfstück ist ein Zuführbehälter angebracht, der lösbar mit dem Rohr verbunden ist, damit das Rohr von unten her in die Öffnung eingesetzt werden kann. Der Zuführbehälter, der eine größere Breite hat als das Rohr, wird anschließend auf dem oberen Rohrende befestigt.

Der Zuführbehälter braucht nicht als Vorratsgefäß ausgebildet zu sein. Zweckmäßigerweise handelt es sich um einen säulenförmigen Behälter, dem die Suspension aus Trägersubstanz und Biokatalysator kontinuierlich zugeführt wird.

Die gesamte Vorrichtung sollte in situ sterilisierbar sein. Das Innere der Vorrichtung, einschließlich des Auffanggefäßes, des Zuführbehälters und der Druckgasleitung, ist gegen die Umgebung keimdicht abgeschlossen, wobei der Abschluß der Druckgasleitung durch ein Sterilfilter erfolgt. Zur Sterilisierung kann Wasser oder eine andere Flüssigkeit, die im Auffanggefäß enthalten ist, durch Beheizung zum Verdampfen gebracht werden. Durch den Dampf wird auch die Tropfvorrichtung, der Zuführbehälter und der Druckgaskanal sterilisiert, wobei der Dampf durch das Sterilfilter entweicht. Solche Hohlräume, die von der Dampfströmung nicht erfaßt werden, sind über eine ein Ventil enthaltende Zweitleitung mit dem Druckgaseinlaß bzw. mit dem Sterilfilter verbunden, so daß bei geöffnetem Ventil auch diese Bereiche durchströmt und sterilisiert werden.

Andererseits kann die Vorrichtung auch im Autoklaven sterilisiert werden, indem sie von dem Auffanggefäß demontiert und in einen Autoklaven eingebracht wird.

Im folgenden wird unter Bezugnahme auf die Zeichnungen ein Ausführungsbeispiel der Erfindung näher erläutert.

Es zeigen:

Fig. 1    eine Seitenansicht der Vorrichtung, teilweise geschnitten,

Fig. 2    eine Seitenansicht der von dem Auffanggefäß getrennten Vorrichtung in vergrößertem Maßstab und

Fig. 3    in vergrößertem Maßstab die Einzelheit III aus Fig. 2.

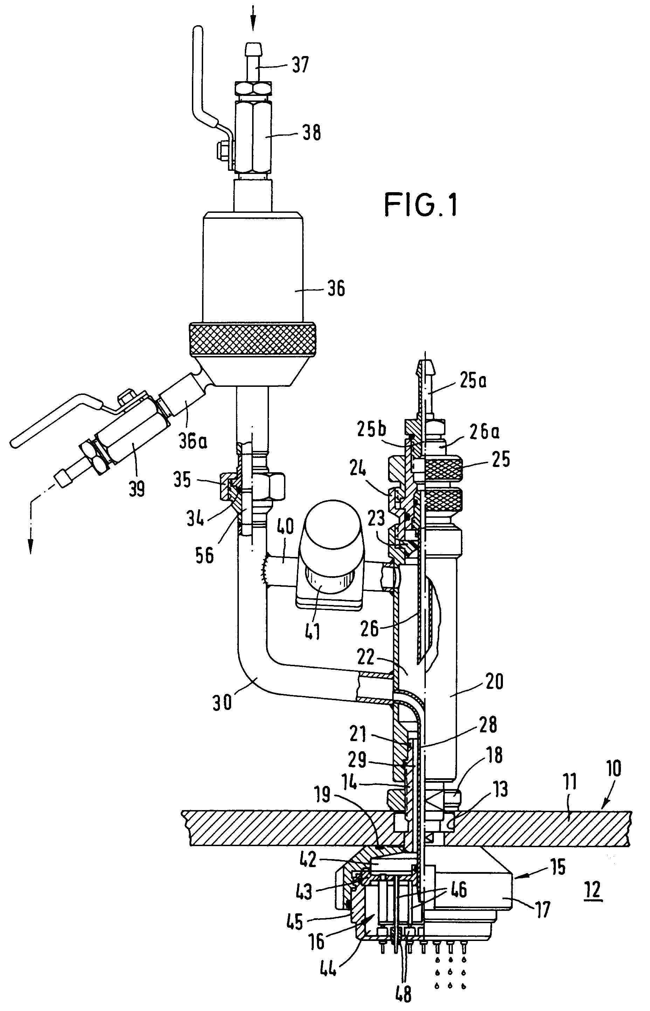

In Fig. 1 ist die Oberwand 11 bzw. der Deckel eines z.B. aus Edelstahl bestehenden Auffanggefäßes 10 dargestellt, dessen Innenraum 12 gegen die Umgebung keimdicht und druckdicht abgeschlossen ist. In der Oberwand 11 des Auffanggefäßes 10 befindet sich eine abgestufte Öffnung 13, bei der es sich um eine Standardöffnung von nur 19 mm Durchmesser handeln kann, die mit einem Schraubstopfen abdichtend verschlossen werden kann.

Von unten her ist in die Öffnung 13 das Rohr 14 eines Kopfstücks 15 eingesetzt, dessen Durchmesser größer ist als derjenige der Öffnung 13. Das Kopfstück 15 enthält die noch zu erläuternde Tropfvorrichtung 16, die in einem nach Art einer Glocke mit nach unten gerichteter Öffnung ausgebildeten Gehäuse 17 untergebracht ist. Mit diesem Gehäuse 17 ist das Rohr 14 fest verbunden. Dieses Rohr ragt über die Oberseite der Oberwand 11 hinaus auf und es hat ein Außengewinde, auf das eine Spannvorrichtung 18 in Form einer Mutter aufgeschraubt ist. Wenn diese Mutter auf dem Gewinde festgezogen wird, zieht sie das Kopfstück 15 gegen die Unterseite der Oberwand 11. Eine Ringdichtung 19 zwischen der Oberseite des Kopfstücks 15 und der Unterseite der Oberwand 11 dichtet den Durchgang des Rohres 14 durch die Öffnung 13 ab.

Auf das Außengewinde des Rohres 14 ist das untere Ende eines Zuführbehälters 20 aufgeschraubt, in welchem das obere Ende des Rohres 14 hineinragt. Die Rohreinführung ist mit einer Ringdichtung 21 abgedichtet.

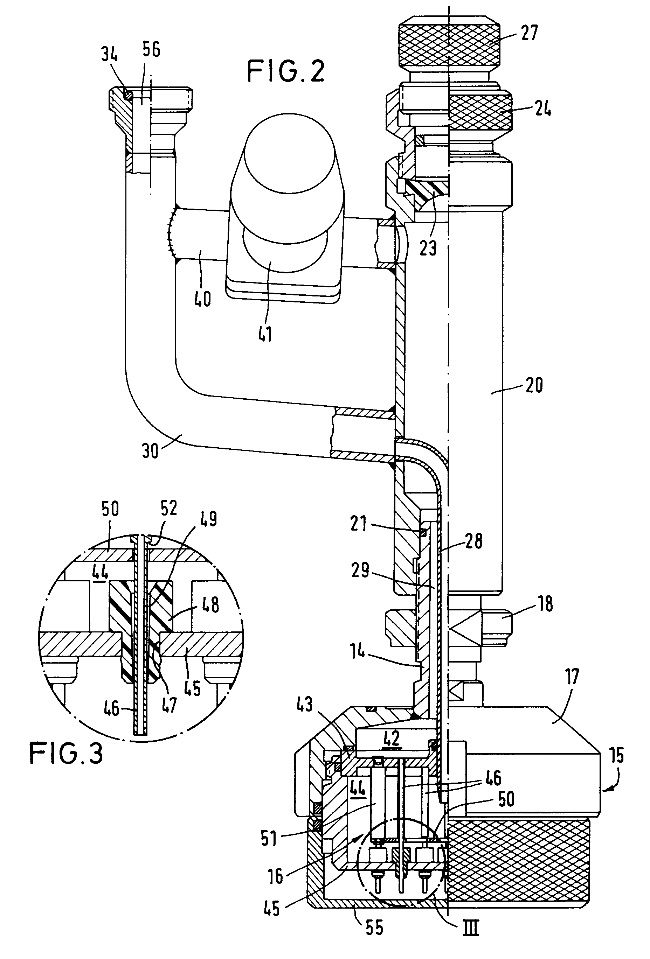

Der Zuführbehälter 20 ist rohrförmig ausgebildet. Er enthält eine Zuführkammer 22, die am oberen Ende durch eine aus Elastomermaterial bestehende Membran 23 abdichtend abgeschlossen ist. Der Rand dieser Membran wird durch ein in das obere Ende des Zuführbehälters 20 eingeschraubtes Klemmstück 24 festgespannt. Das Klemmstück 24 weist ein Innengewinde auf, in das entweder der Halter 26a einer hohlen Anstechnadel 26 mit einer Anschlußmutter 25 eingeschraubt werden kann (Fig. 1) oder ein Stopfen 27 (Fig. 2) zum abdichtenden Verschließen der oberen Öffnung des Zuführbehälters. Der Halter 26a weist an seinem rückwärtigen Ende einen Schlauchanschluß 25a auf, mit einem Längskanal 25b, der mit dem Längskanal der Anstechnadel 26 in Verbindung steht. An den Schlauchanschluß 25a wird ein Schlauch angeschlossen, der zu einem (nicht dargestellten) Vorratsgefäß führt, welches eine flüssige Trägersubstanz und den darin suspergierten Biokatalysator enthält. Die Suspension wird dem Zuführbehälter 20 unter Druck durch z.B. Überlagerungs- oder Pumpendruck mit vorgegebener Zuführrate zugeführt. Sie gelangt dadurch in die Zuführkammer 22. Nach Beendigung der Immobilisierung kann die Transferleitung zwischen Schlauchanschluß 25a und nicht dargestelltem Vorratsgefäß keimdicht verschlossen und das Vorratsgefäß abgekoppelt werden.

Durch das Rohr 14 verläuft koaxial ein rohrförmiger Druckgaskanal 28, durch den Druckgas, z.B. sterile Luft, in das Kopfstück 15 eingeleitet wird. Der Druck-Zuführbehälter 20, der auch beheizbar gestaltet werden kann, erstreckt sich nach unten über das Rohr 14 hinaus in das Kopfstück hinein. Der Druckgaskanal 28 ist von dem ringförmigen Flüssigkeitskanal 29 umgeben, der mit dem der Zuführkammer 22 in Verbindung steht und der ebenfalls in das Kopfstück 15 hineinführt. Das obere Ende des Druckgaskanals 28 ist zur Seitenwand des Zuführbehälters 20 hin abgebogen und an eine externe Rohrleitung 30 angeschlossen, die über eine mit einer Dichtung 34 versehene lösbare Schraubverbindung 35 mit einem Sterilfilter 36 verbunden ist. Das Sterilfilter 36 ist ein Bakterienfilter, das die Druckluft filtert, die ihm über den Druckluftanschluß 37 mit einstellbarem Druck zugeführt wird. Diese Druckluft ist über ein Ventil 38 absperrbar. Das untere Ende des Sterilfilters 36 weist einen Kondensatablauf 36a auf, an den ein Ventil 39 angeschlossen ist, über das bei der Sterilisation des Gerätes Kondensat abgelassen wird, das sich in einer unteren Auffangwanne des Sterilfilters 36 angesammelt hat.

Vom oberen Ende des Zuführbehälters 20 erstreckt sich unmittelbar unter der Membran 23 eine Bypass-Leitung 40, die ein handbetätigbares Ventil 41 enthält, zu der Rohrleitung 30. Dieses Ventil 41 wird nur zu Sterilisationszwecken geöffnet, wie nachfolgend noch erläutert wird.

Das Kopfstück 15 enthält im oberen Bereich des Gehäuses 17 eine Flüssigkeitskammer 42, in die der Flüssigkeitskanal 29 einmündet und die nach unten hin durch eine Trennwand 43 begrenzt ist. Durch eine Öffnung in der Mitte der Trennwand 43 führt der Druckgaskanal 28 abdichtend hindurch in eine Druckkammer 44 hinein. Die Druckkammer 44 ist durch einen becherförmigen Boden 45 begrenzt, der mit einem Außengewinde in ein Innengewinde des Gehäuses 17 eingeschraubt ist und der das Innere des Gehäuses abdichtend verschließt.

Die Trennwand 43 weist zahlreiche Bohrungen auf, in die die oberen Enden von Auslaufrohren 46 eingesetzt sind. Diese Enden stehen mit der Flüssigkeitskammer 42 in Verbindung. Die Auslaufrohre 46 ragen durch die Druckgaskammer 44 hindurch und sie verlaufen durch Öffnungen des Bodens 45. Die unteren Enden der Auslaufrohre 46 stehen aus dem Kopfstück 15 heraus vor.

Wie Fig. 3 zeigt, sind im Boden 45 Öffnungen 47 vorgesehen, in denen Kunststoffbuchsen 48 zum Zentrieren der Auslaufrohre 46 sitzen. Diese Kunststoffbuchsen 48 weisen jeweils einen sich nach oben konisch erweiternden Kanal auf, der eine Durchtrittsöffnung 49 für den Durchtritt von Gas bildet. Die Kunststoffbuchsen 48 umschließen somit die Auslaufrohre nicht eng, sondern zwischen beiden bestehen die rechteckigen Durchtrittsöffnungen 49, durch die Druckgas nach unten hin ausströmen kann, um jeweils von der aus dem Auslaufrohr ausfließenden Flüssigkeit Tropfen abzureißen. Diese Tropfen fallen in die im Auffanggefäß 10 befindliche Vernetzerlösung bzw. das Umfällbad, wo sie zu Katalysatorperlen erstarren.

Die Druckgaskammer 44 enthält einen plattenförmigen Halter 50, der über Abstandhalter 51 an der Trennwand 43 aufgehängt ist und der Zentrierungsöffnungen für den Durchtritt der Auslaufrohre 46 enthält. Auf dem Halter 50 stützen sich die Auslaufrohre 46 jeweils mit einer Schulter 52 ab (Fig. 3).

Die beschriebene Vorrichtung zur Herstellung von Katalysatorperlen arbeitet wie folgt:
Eine Trägersubstanz, beispielsweise eine viskose Polyelektrolytlösung (Alginat, Pektinat, Carboxymethylcellulose, Carrageenan, Furcellaran, Cellulosesulfat, Chitosan usw.), eine gelierfähige Polymerlösung (Curdlan, Agar, Agarose, Gelatine usw.) oder eine nichtwäßrige Polymerlösung (Celluloseacetat, Polystyrol, Eudragit usw.) wird mit den Enzymen, Zellorganellen oder ganzen Zellen gemischt. Diese Mischung wird in ein Vorratsgefäß gegeben. Die Mischung gelangt durch Überlagerungs- oder Pumpendruck über den Schlauchanschluß 25a und die Anstechnadel 26 in die Zuführkammer 22. Unter diesem Druckeinfluß gelangt die Mischung durch den Flüssigkeitskanal 29 in die Flüssigkeitskammer 42 der Tropfvorrichtung 16 und von dort in die Auslaufrohre 46. Beim Austreten aus den unteren Enden der Auslaufrohre 46 wird die Flüssigkeit durch die aus den Durchtrittsöffnungen 49 (Fig. 3) austretenden Druckgasstrahlen in Tropfenform abgerissen. Diese Tropfen fallen in die Vernetzerlösung bzw. das Umfällbad und erstarren zu Katalysatorperlen, in denen der Biokatalysator immobilisiert ist.

Bei der beschriebenen Arbeitsweise wird dem Druckgasanschluß 37 Druckgas zugeführt, wobei das Ventil 38 geöffnet und das Ventil 39 gesperrt ist. Das Druckgas gelangt über das Sterilfilter 36 und die Anschlußvorrichtung 35 zum Druckgaseinlaß 56 und von dort über die Rohrleitung 30 und den Druckgaskanal 28 in die Druckkammer 44, aus der es durch die Ausströmöffnungen 49 austritt.

Wenn die beschriebene Vorrichtung in situ, also auf dem Auffanggefäß 10, sterilisiert werden soll, wird eine in dem Auffanggefäß enthaltene Flüssigkeit, bei der es sich z.B. um die Vernetzerlösung oder das Umfällbad handeln kann, durch Erwärmung verdampft und beide Ventile 38 und 41 werden geöffnet. In diesem Zustand ist die Anstechnadel 26 nicht im Gerät enthalten. Der Dampf im Auffanggefäß 10 entweicht durch den Überdruck durch die Auslaufrohre 46 hindurch in die Flüssigkeitskammer 42 und durch den Flüssigkeitskanal 29 in die Zuführkammer 22. Außerdem gelangt Dampf durch die Durchtrittsöffnungen 49 in die Druckkammer 44 und den Flüssigkeitskanal 28, die dadurch sterilisiert werden. Der Dampf strömt weiter durch die Rohrleitung 30 und das Sterilfilter 36, die ebenfalls sterilisiert werden. Im Sterilfilter 36 kondensiert ein Teil des Dampfs. Das entstehende Kondensat wird über das Ventil 39 abgeleitet.

Bei der bisher beschriebenen Art der Dampfkondensierung würde sich in der Zuführkammer 22, die am oberen Ende durch die Membran 23 abgedichtet ist, eine Tasche ergeben, in der keine Dampfströmung vorhanden ist. Um auch den oberen Bereich der Zuführkammer 22 durch strömenden Dampf zu sterilisieren, ist die Bypass-Leitung 40 vorgesehen. Der Dampf strömt über das geöffnete Ventil 41 von der Zuführkammer 22 in die Rohrleitung 30. Für den eigentlichen Betrieb der Vorrichtung wird das Ventil 41 wieder geschlossen. Anstelle eines manuell zu betätigenden Ventils kann auch ein Rückschlagventil oder ein steuerbares Ventil benutzt werden.

Sämtliche Teile der dargestellten Vorrichtung, mit Ausnahme der Membran 23, der Dichtringe und der Kunststoffbuchsen 48, bestehen aus Edelstahl, also einem Material, das leicht mit Dampf zu sterilisieren ist, so daß die Sterilisierung in situ erfolgen kann. Für den Fall, daß die gesamte Einheit nicht fest in einem Auffanggefäß montiert werden soll, kann die Sterilisation auch in einem Autoklaven durchgeführt werden. Hierbei kann dann das relativ aufwendige Sterilfilter 36 durch ein einfacheres Sterilfilter ersetzt werden. Vor der Sterilisation im Autoklaven wird das Kopfstück 15 in die Zuführkammer 20 eingeschraubt und diese durch die Membran 23, die Druckmutter 24 und den Blindstopfen 27 keimdicht verschlossen. Außerdem wird das Kopfstück 15 durch die Kappe 55 keimdicht verschlossen.

**Patentansprüche**

1. Vorrichtung zur Herstellung von Biokatalysatorperlen, mit

   einem mit einer Öffnung (13) versehenen Auffanggefäß (10) zur Aufnahme eines Umfällbades oder einer Vernetzerlösung und

   einer an eine Zuführeinrichtung anschließbaren Tropfvorrichtung (16), die in das Auffanggefäß (10) hineinragende Auslaufrohre (46) aufweist, welche durch eine Druckkammer (44) hindurchgehen, wobei die Druckkammer (44) die Auslaufrohre (46) umgebende Durchtrittsöffnungen (49) aufweist,

   **dadurch gekennzeichnet ,**

   daß die Tropfvorrichtung (16) im Inneren des Auffanggefäßes (10) in einem Kopfstück (15) enthalten ist, dessen Breite größer ist als diejenige der Öffnung (13) des Auffanggefäßes und das ein unter Abdichtung durch die Öffnung (13) hindurchgehendes Rohr (14) mit Druckgaskanal (28) und Flüssigkeitskanal (29) aufweist.

2. Vorrichtung nach Anspruch 1, dadurch gekennzeichnet, daß an dem Rohr (14) eines das Kopfstück (15) gegen den Rand der Öffnung (13) ziehende Spanneinrichtung (18) angreift.

3. Vorrichtung nach Anspruch 1 oder 2, dadurch gekennzeichnet, daß auf dem Rohr (14) ein Zuführbehälter (20) angebracht ist, der eine Zuführkammer (22) aufweist.

4. Vorrichtung nach Anspruch 3, dadurch gekennzeichnet, daß der Druckgaskanal (28) Bestandteil des Zuführbehälters (20) ist und aus der Zuführkammer (22) seitlich herausführend gestaltet ist.

5. Vorrichtung nach Anspruch 4, dadurch gekennzeichnet, daß ein Druckgaseinlaß (56) über eine ein Ventil (41) enthaltende Bypass-Leitung (40) mit der Zuführkammer (22) in Verbindung steht.

6. Vorrichtung nach einem der Ansprüche 1-5, dadurch gekennzeichnet, daß der Druckgaskanal (28) koaxial in das Kopfstück (15) hineinführend und dort abdichtend durch eine die Auslaufrohre (46) haltende Trennwand (43) hindurch in die Druckkammer (44) hineinragend gestaltet ist.

7. Vorrichtung nach einem der Ansprüche 1-6, dadurch gekennzeichnet, daß eine die Druckkammer (44) abschließende Wand (45) Durchtrittsöffnungen (47) für die Auslaufrohre (46) enthält, wobei die Auslaufrohre in den Durchtrittsöffnungen (47) innerhalb von Kunststoffbuchsen (48) durch die rechteckigen Ausströmöffnungen (49) zentriert sind.

8. Vorrichtung nach einem der Ansprüche 3-6, dadurch gekennzeichnet, daß der Zuführbehälter (20) durch eine durchstechbare Membran (23) abgeschlossen ist, durch die eine Anstechnadel (26) stoßbar ist, welche mit einem Behälter verbunden ist, der in einer Trägerflüssigkeit suspendierten Biokatalysator enthält.

9. Vorrichtung nach einem der Ansprüche 1-8, dadurch gekennzeichnet, daß das Kopfstück (15) ein geschlossenes Gehäuse (17) aufweist, aus dem die Enden der Auslaufrohre (46) herausragen und daß eine die Enden der Auslaufrohre keimdicht umschließende, eine sterile Handhabung nach Sterilisation beim Einsatz außerhalb des Auffanggefäßes (10) ermöglichende Kappe (55) vorgesehen ist.

FIG.1

EP 0 515 994 A2

FIG.2

FIG.3

7